# EUROPEAN PATENT APPLICATION

(11) **EP 4 674 396 A1**
(43) Date of publication of application: **07.01.2026**
(21) Application number: 24186011.3
(22) Date of filing: 02.07.2024
(51) Int. Cl.: A61F 13/00, A61F 13/0206, A61B 5/01, A61B 5/00

(54) **AN ADHESIVE SENSOR STRIP**

(71) Applicant: Mölnlycke Health Care AB, 431 21 Mölndal (SE)
(72) Inventor: JAKOBSSON, Conny, 443 38 LERUM (SE); FLACH, Niclas, 441 35 ALINGSÅS (SE)
(74) Representative: Kransell & Wennborg KB

(57) **Abstract**

The present disclosure generally relates to adhesive sensor strip (1) for use in conjunction with a medical dressing (2), wherein the sensor strip (1) has a lateral (x) and a longitudinal (y) extension; the lateral (x) extension being larger than the longitudinal (y) extension, wherein the sensor strip (1) comprises a first portion (3) and a second portion (4); the lateral (x) extension of the first portion (3) being from 50 to 350 mm, wherein the lateral (x) extension of the second portion (4) is from 15 to 100 mm, wherein the first portion (3) comprises a plurality of sensors (5a-d) configured to monitor at least one parameter indicative of infection, and wherein the second portion (4) comprises an electrical connector (6) being connected to the plurality of sensors (5a-c) and being configured for connection with a separate processing device

The medical dressing also relates to a medical dressing assembly (14) comprising the adhesive sensor strip (1) and a medical dressing (2).

## Description

### TECHNICAL FIELD

The present disclosure generally relates to an adhesive sensor strip for use in conjunction with a medical dressing, wherein the sensor strip has a lateral (x) and a longitudinal (y) extension; the longitudinal (y) extension being larger than the lateral (x) extension, wherein the sensor strip comprises a first portion and a second portion. The first portion comprises a plurality of sensors configured to monitor at least one parameter indicative of infection. The present disclosure also relates to a medical dressing assembly comprising the adhesive sensor strip and a medical dressing.

### BACKGROUND

Surgical site infections (SSI) are a significant concern in post-operative care. Surgical site infections can lead to severe complications, prolonged recovery times, increased medical costs, and in extreme cases, patient mortality.

Early detection of SSIs is critical to mitigate these complications. Traditional methods of monitoring SSIs often involve visual inspection and manual assessment. However, these methods can be imprecise and inadequate, especially when the wound or incision is covered by a dressing, making it difficult to observe changes that may indicate an infection.

Recent technological advancements have introduced various types of sensors capable of monitoring parameters indicative of infection, such as temperature, humidity, and biochemical markers. These sensors provide a more accurate and continuous means to detect early signs of infection. However, integrating these sensors into a medical dressing is associated with several challenges.

For example, precise placement of the sensors with respect to the incision (or wound) to ensure accurate readings may be a challenge. The sensors must be positioned correctly to monitor the relevant parameters effectively. Furthermore, the arrangement of the sensors should desirably not interfere with the wound-healing process.

Medical dressings embedded with electronic components also pose significant challenges when it comes to disposal. Such dressings are classified as hazardous waste and require careful separation of the electronic components to ensure proper disposal and recycling. This process is generally labor-intensive and costly.

Furthermore, a dressing embedded with sensors is typically complex and expensive to manufacture. Accordingly, for economical and practical reasons it is desirable that the sensors remain on the skin for a prolonged time, reducing the frequency of dressing changes and ensuring continuous monitoring without interruption.

In view of this, it is desirable to provide improvements in the field of sensing devices to secure early and reliable detection of SSIs, while also alleviating the problems described hereinbefore.

### SUMMARY

In view of the above-mentioned problems, it is an object of the present disclosure to provide improvements in the field of sensing devices and to provide a simplified, cost-efficient, and more sustainable means to monitor SSIs.

In a first aspect, there is provided an adhesive sensor strip for use in conjunction with a medical dressing, wherein the sensor strip has a lateral (x) and a longitudinal (y) extension; the lateral (x) extension being larger than the longitudinal (y) extension, wherein the sensor strip comprises a first portion and a second portion; the lateral (x) extension of the first portion being from 50 to 350 mm, wherein the lateral (x) extension of the second portion is from 15 to 100 mm, wherein the first portion comprises a plurality of sensors configured to monitor at least one parameter indicative of infection, and wherein the second portion (4) comprises an electrical connector being connected to the plurality of sensors and being configured for connection with a separate processing device.

The present disclosure is based on the realization that the adhesive sensor strip, when used in conjunction with a medical dressing, significantly alleviates the problems associated with conventional wound dressings comprising embedded sensors and electronic components.

The adhesive sensor strip allows for a controlled application onto a surgical incision and a precise alignment of the sensors with respect to the incision. The sensor strip is to be used in conjunction with a separate medical dressing.

Accordingly, in a first step, the medical personnel may arrange the sensor strip onto an incision of a patient's skin and precisely position the sensors with respect to the incision. Subsequently, a separate medical dressing may be arranged on top of the sensor strip and attached to the strip and to the skin of a patient.

In this regard, the adhesive sensor strip comprising the plurality of sensors may be firmly anchored to the incision site and to the skin of the patient and be held in close skin-contact during use.

The maximum lateral (x) extension; i.e. length of the first portion of the adhesive sensor strip corresponds to the maximum (x) lateral extension; i.e. length of an absorbent pad of the medical dressing. Accordingly, the plurality of sensors are arranged in the area where wound exudate is continuously transferred from the wound or incision site towards the backing layer (from which the wound exudate evaporates).

The medical dressing is arranged onto the sensor strip such that at least a part of the second portion (which comprises the electrical connector) extends beyond a peripheral edge of the medical dressing.

The electrical connector may be connected to the sensors by an electrical circuit. The electrical connector is configured to be connected to a separate processing device to receive and evaluate the data from the sensors. The processing device may comprise a power source, a processing unit and means to communicate the data with an electronic device. The arrangement of the electrical connector at the second portion of the sensor strip allows for the processing device to be coupled to the sensor strip in a location remote from the incision and outside the peripheral edges of the medical dressing.

This facilitates battery changes, data retrieval, and other maintenance activities without disturbing the wound site or compromising the integrity of the strip/dressing. By having the electrical connector positioned away from the wound site and the dressing, there is also less "bulk" and discomfort for the patient.

In addition, the medical dressing may be detached from the sensor strip (and the skin), when saturated, i.e. when the dressing has reached its full capacity, and replaced with a new one, while keeping the sensor strip attached to the skin. The wear time of the sensor strip may thus be significantly prolonged compared to conventional dressings comprising integrated sensors. Since the medical dressing used in conjunction with the sensor strip is typically void of sensors and electronic components, the costs associated with several dressing changes can be significantly reduced. The sensor strip remains on the skin and may provide continuous (non-interrupted) real-time data and monitoring of the wound or incision.

As mentioned hereinbefore, the medical dressing is preferably void of sensors and electronic components. In this regard, the major parts of the used materials can be handled as regular medical waste, which significantly reduces the burden on hazardous waste management.

The sensor strip of the present disclosure may be used with various types of medical dressings. Medical dressings come in a huge variety of sizes and shapes. The length, i.e. lateral (x) extension of the strip may therefore vary. The lateral (x) extension of the first portion may vary depending on the size of the incision and the size (or length) of the medical dressing. The lateral (x) extension of the second portion is typically the same regardless of the size of the medical dressing or the incision.

Typically, the second portion is void of sensors.

This is beneficial to minimize interference with the electrical connector and to keep the sensors in an area where they are better protected from becoming damaged during use (below the medical dressing). This is also beneficial from a manufacturing point of view.

The sensor strip of the present disclosure is effective in preventing and detecting SSIs, and offers an environmentally sound, cost-effective, and user-friendly solution for patients and medical personnel.

The plurality of sensors is configured to monitor at least one parameter indicative of infection. Accordingly, accurate and timely detection of SSIs can be accomplished.

Typically, the plurality of sensors is selected from the group consisting of temperature sensors, pH sensors, humidity sensors, biochemical sensors, and/or optical sensors. Preferably, the plurality of sensors is temperature sensors.

Temperature sensors can detect localized increases in temperature, which may indicate inflammation or infection. A rise in temperature is often one of the first signs of infection.

The pH level of wound exudate typically changes in response to infection. Hence, pH sensors can provide early warning signs of infection by detecting shifts in the wound environment's acidity or alkalinity.

Humidity sensors measure the moisture level in and around the incision or wound. Excessive moisture can create an environment stimulating bacterial growth and infection. Monitoring humidity may secure that the wound environment is kept at an optimal level for healing.

Biochemical sensors can detect specific markers or pathogens associated with infection. These sensors may e.g. be configured to detect bacterial toxins, inflammatory cytokines, or other biochemical substances indicative of infection.

Optical sensors may be used to detect changes in the wound's appearance, such as color changes or the presence of pus. These sensors can provide visual confirmation of infection and monitor the wound healing progress.

In exemplary embodiments, the plurality of sensors is arranged in a linear sequence.

Surgical incisions are typically substantially linear. The arrangement of the sensors in a linear sequence, i.e. along a straight line, thus secure that the sensors are consistently positioned along the length of an incision. This reduces the likelihood of misalignment, which can lead to inaccurate readings and potentially delayed infection detection.

The sensor readings along the incision help medical personnel to quickly identify trends or anomalies. For example, a gradual increase in temperature readings along the length of the incision may indicate the spread of an infection, which may prompt timely intervention.

While the linear sequence arrangement is optimal for linear surgical incisions, it may of course, be used with other types of wounds by arranging the line of sensors strategically along the area of interest.

In exemplary embodiments, at least the first portion comprises a plurality of perforations.

Accordingly, wound exudate may pass through the strip and be absorbed by the overlying medical dressing. In this regard, exudate is prevented from accumulating at the wound site, which may cause maceration of the skin and delay the wound healing process. Furthermore, the sensors are less likely to be obstructed or impaired by exudate buildup.

In exemplary embodiments, the second portion is divided into an intermediate section and a terminal section; the intermediate section being arranged between the first portion and the terminal section of the sensor strip, wherein the electrical connector is arranged in the terminal section.

A medical dressing typically comprises a backing layer (top layer), an adhesive skin-contact layer and an absorbent pad arranged between the backing layer and the adhesive-skin contact layer.

The backing layer, absorbent pad and adhesive skin-contact layer may be co-extensive in length and width. Alternatively, the absorbent pad has a smaller surface area than the backing layer and the adhesive skin-contact layer. The backing layer and the adhesive skin-contact layer are thus arranged to surround the edges of the absorbent pad to form a border portion. Such a "border dressing" is typically preferred for post-operative incisions.

In embodiments where the medical dressing is a border dressing, as described hereinabove, the absorbent pad is arranged to cover the first portion of the adhesive sensor strip during use. As mentioned hereinbefore, the maximum lateral (x) extension of the first portion corresponds to the maximum lateral (x) extension of the absorbent pad. The border portion of the medical dressing is arranged to cover the intermediate section of the second portion of the sensor strip during use. The terminal section comprising the electrical connector is arranged to extend beyond the border portion, i.e. beyond a peripheral edge of the medical dressing.

This construction secures that the first portion comprising the sensors is well protected under the medical dressing. A consistent contact with the skin of a patient is thus provided such that reliable and accurate sensor readings are accomplished. The terminal section, arranged outside the peripheral edge of the dressing, allows for the electrical connector to be easily accessed and managed during use (and during dressing changes). Accordingly, the processing device does not interfere with the incision during use. Furthermore, batteries may be changed, the processing device may be connected and disconnected from the sensor strip while the sensor strip and the medical dressing remain attached to the skin.

The adhesive sensor strip may comprise a polymeric film and an adhesive skin-contact layer; the adhesive skin-contact layer preferably comprising a silicone-based adhesive.

The polymeric film is desirably thin and pliable such that it can stretch and conform to the movement of a wearer. The adhesive skin-contact layer secures that the sensor strip remains attached to the skin during use.

A silicone-based adhesive is gentle and non-irritating to the skin. It is a soft and flexible adhesive which conforms well to the body contours without causing trauma upon removal.

In exemplary embodiments, the sensor strip further comprising a leakage-protective coating arranged on at least the intermediate section of the second portion.

This is to prevent wound exudate migrating from the absorbent pad towards the border portion of a medical dressing. If wound exudate leaks from the absorbent area to the adhesive border portion, this may cause the border to detach from the skin, thereby compromising the wear time of the medical dressing (and the sensor strip). The leakage-protective coating acts as a barrier to prevent wound exudate from migrating towards the border portion. In this regard, the border portion, the second portion of the sensor strip as well as the skin underlying the border portion are kept dry, which is important for proper skin-adhesion.

The adhesive sensor strip has a top side and a bottom side. The bottom side is arranged to contact the skin of a patient during use. The top side is arranged to contact an overlying medical dressing during use. The leakage-protective coating is typically arranged on the top side of the sensor strip (in the intermediate section).

In exemplary embodiments, the adhesive sensor strip further comprises at least one printed marking indicating where a peripheral edge of the medical dressing should be arranged on the sensor strip during use.

The printed markings guide the medical personnel to a correct positioning of the medical dressing. Accordingly, the dressing can be arranged to cover the incision or wound and the first portion of the strip. The second portion can be correctly arranged with respect to the peripheral dressing edge. Hence, the application procedure for medical personnel is simplified and the likelihood of incorrect placement is eliminated.

In exemplary embodiments, the at least one printed marking is arranged at the interface of the intermediate and terminal sections.

This is particularly beneficial in embodiments where the medical dressing is a border dressing. The border portion of the medical dressing can thus be correctly positioned with respect to the intermediate section of the adhesive sensor strip and such that the terminal section (comprising the electrical connector) is arranged outside the peripheral dressing edge.

According to another aspect, there is provided a medical dressing assembly comprising the adhesive sensor strip as described hereinbefore, and a medical dressing, wherein the medical dressing is arranged on top of the first portion of the sensor strip during use; the medical dressing being defined by peripheral edges, and wherein at least the electrical connector of the second portion is arranged to extend beyond one of the peripheral edges of the medical dressing.

The medical dressing may comprise a backing layer, an adhesive skin-contact layer, and an absorbent pad arranged between the backing layer and the adhesive skin-contact layer; the absorbent pad being defined by pad edges, wherein the backing layer and the adhesive-skin-contact layer are arranged to extend beyond the pad edges to form a border portion surrounding the absorbent pad.

The medical dressing may have a lateral (x) extension and a longitudinal (y) extension; the absorbent pad having a maximum lateral (x) extension corresponding to the maximum lateral (x) extension of the first portion of the sensor strip, wherein the border portion has a width corresponding to the maximum lateral (x) extension of the intermediate section of the second portion of the sensor strip.

In embodiments where the backing layer, absorbent pad, and adhesive skin-contact layer of the medical dressing are co-extensive, the entire part of the second portion of the sensor strip extends beyond one of the peripheral edges of the medical dressing. In contrast, for a "border dressing", as defined hereinbefore, only the terminal section of the second portion of the sensor strip extends beyond one of the peripheral edges of the medical dressing. The intermediate section is "covered" by the border portion of the dressing.

Preferably, the adhesive skin-contact layer comprises a plurality of perforations in the area underlying the absorbent pad but is void of perforations in the area forming the border portion.

The perforations underlying the absorbent pad secure an efficient liquid acquisition, and liquid handling in the area of the dressing where this is desirable, i.e. in the absorbent pad.

The non-perforated border portion prevents leakage, i.e. migration of wound exudate from the absorbent pad to the border portion. The lack of perforations in the border portion thereby enhances the adherence of the dressing to the skin such that the dressing may stay on the skin for prolonged periods.

In exemplary embodiments, the medical dressing further comprises a processing device configured for connection with the electrical connector.

The electrical connector on the sensor strip provides a conductive interface that links the sensors to the processing device. This connection ensures that the data collected by the sensors is accurately transmitted to the processing device for evaluation. The processing device processes this data and may transmit the data to an external system, e.g. an electronic device for further analysis and/or for alerting medical personnel of an ongoing wound infection.

The medical dressing is preferably void of any sensors and of any electrical components.

This is beneficial from a waste management, cost efficiency, and manufacturing perspective.

Further features of, and advantages with, the present disclosure will become apparent when studying the appended claims and the following description. The skilled addressee realizes that different features of the present disclosure may be combined to create embodiments other than those described in the following, without departing from the scope of the present disclosure.

### BRIEF DESCRIPTION OF DRAWINGS

The various aspects of the present disclosure, including its particular features and advantages, will be readily understood from the following detailed description and the accompanying drawings, in which:
Figure 1 schematically illustrates an adhesive sensor strip, seen from the top side, according to an exemplary embodiment of the present disclosure. Figure 1 also illustrates where a medical dressing is to be arranged on the adhesive sensor strip.
Figure 2 schematically illustrates a medical dressing assembly according to an exemplary embodiment of the present disclosure.
Figure 3 is a cross-sectional view of the medical dressing assembly illustrated in figure 2.
Figure 4 schematically illustrates a partially split view of a medical dressing for use in conjunction with the adhesive sensor strip or forming part of the medical dressing assembly according to an exemplary embodiment of the present disclosure.

### DETAILED DESCRIPTION

The present disclosure will now be described more fully hereinafter with reference to the accompanying drawings, in which currently preferred embodiments of the present disclosure are shown. The present disclosure may, however, be embodied in many different forms and should not be construed as limited to the embodiments set forth herein; rather, these embodiments are provided for thoroughness and completeness, and fully convey the scope of the present disclosure to the skilled person. Like references refer to like elements throughout.

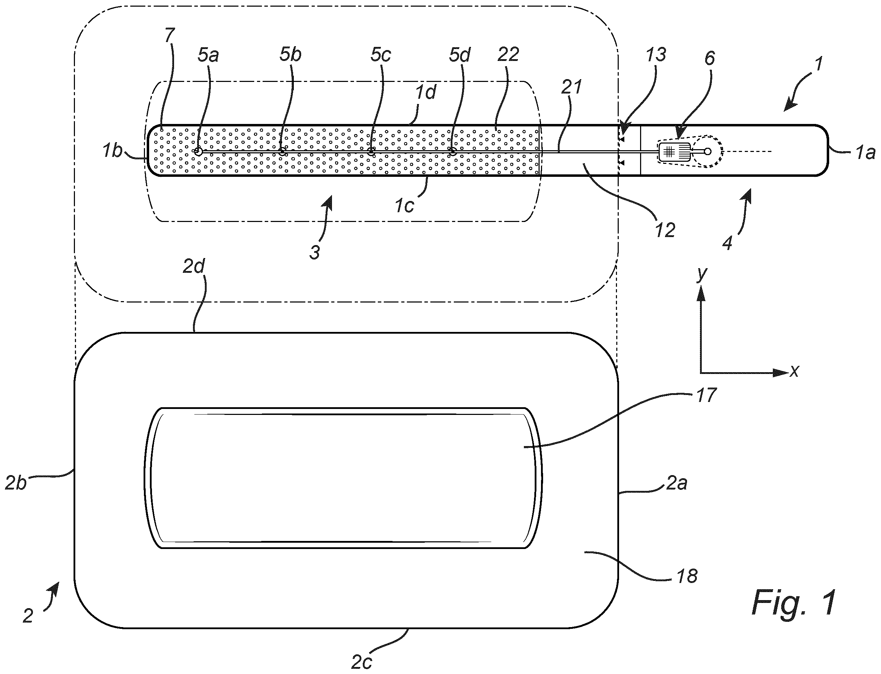

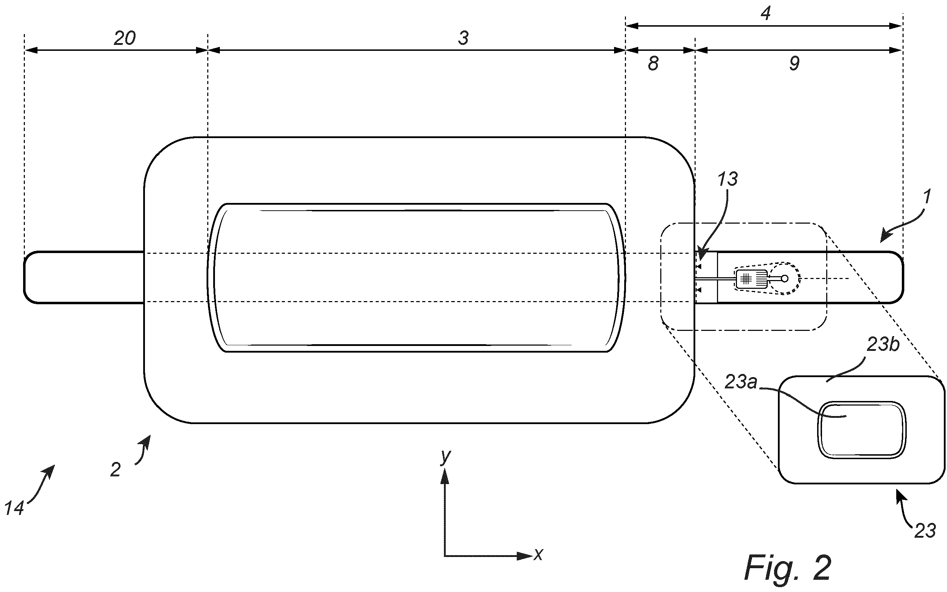

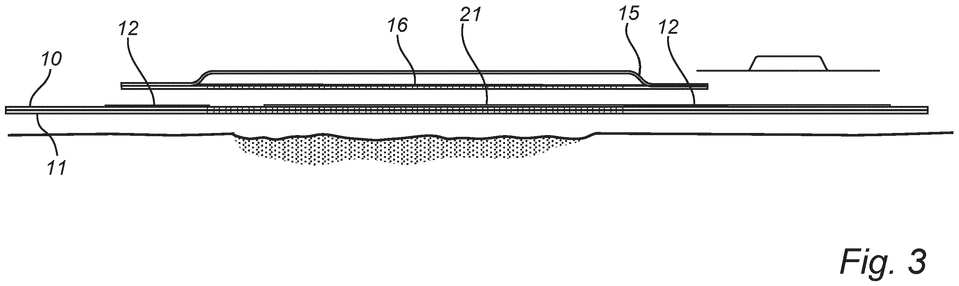

The adhesive sensor strip will now be explained with reference to figures 1, 2, and 3. Figure 1 schematically illustrates an adhesive sensor strip of the present disclosure. Figure 2 schematically illustrates an adhesive sensor strip according to an alternative embodiment when a medical dressing has been arranged on top of the sensor strip. Figure 3 is a cross-sectional view of the adhesive sensor strip and the medical dressing of figure 2.

The adhesive sensor strip 1 has a lateral (x) and a longitudinal (y) extension, wherein the lateral (x) extension is larger than the longitudinal (y) extension. The adhesive sensor strip 1 comprises a first portion 3 and a second portion 4; the maximum lateral (x) extension of the first portion 3 being from 50 to 350 mm, wherein the maximum lateral (x) extension of the second portion 4 is from 15 to 100 mm, wherein the first portion 3 comprises a plurality of sensors 5a-d configured to monitor at least one parameter indicative of infection, and wherein the second portion 4 comprises an electrical connector 6 being connected to the plurality of sensors 5a-c and being configured for connection with a separate processing device.

The "maximum lateral (x) extension" may also be referred to as the length of the adhesive sensor strip. The "maximum longitudinal (y) extension" may also be referred to as the width of the adhesive sensor strip. The lateral (x) extension is perpendicular to the longitudinal (y) extension. The adhesive sensor strip has a rectangular shape.

The maximum lateral (x) extension of the sensor strip may be from 100 to 600 mm.

The maximum longitudinal (y) extension of the sensor strip may be from 10 to 70 mm, e.g. from 15 to 40 mm.

The adhesive sensor strip 1 is used in conjunction with a medical dressing 2, which is arranged on top of the sensor strip 1 during use. As best illustrated in figure 2, the adhesive sensor strip 1 comprises a first portion 3 and a second portion 4. In figure 2, the adhesive sensor strip 1 further comprises a third portion 20. The first portion 3 is arranged between the second 4 and third 20 portions.

As illustrated in figures 1-3, the second portion 4 extends beyond a peripheral edge 2a of the medical dressing 2 during use. The third portion 20, where present, may also extend beyond a peripheral edge of the medical dressing 2 (see figures 2 and 3). Hence, the second portion 4 may extend beyond a first peripheral edge 2a of the medical dressing 2, and the third portion 20 may extend beyond a second, opposing peripheral edge 2b of the medical dressing 2.

This arrangement may be beneficial to facilitate detachment of the medical dressing 2 from the adhesive sensor strip 1 and from the skin surface. If the edge portions (i.e. second and third portions) of the sensor strip would be "covered" by the medical dressing, there is a risk that the sensor strip is removed with the medical dressing when the medical dressing is to be detached form the skin. If both the second 4 and the third 20 portions extend beyond the peripheral edges of the dressing, the medical dressing 2 may be detached and removed from the sensor strip 1 and the skin from either dressing edge (without risking detachment of the sensor strip 1).

Alternatively, the third portion 20 is absent (as illustrated in figure 1), and the first portion is arranged below the medical dressing and being completely covered by the medical dressing. This construction is beneficial to prevent migration of wound exudate from the absorbent pad towards the border portion of the medical dressing.

The second portion 4 comprises the electrical connector 6 and is arranged remote from the incision, the plurality of sensors 5a-d, and the medical dressing 2 during use.

As used herein the term "plurality of sensors" means at least two, preferably at least three sensors. Typically, the adhesive sensor strip comprises from 3 to 10, e.g. from 4 to 8 sensors. The number of sensors may vary depending on the size of the adhesive strip, the size of the incision, and the type of wound or incision.

As used herein, the term "electrical connector" means a conductive interface component configured to establish an electrical connection between the sensors and an external processing device, thereby facilitating the transfer of power and data.

The electrical connector 6 may be connected to the plurality of sensors 5a-d by means of an electrical circuit 21. The electrical circuit 21 transmits signals and power between the sensors and an external processing device (connected to the electrical connector 6).

The electrical circuit 21, the plurality of sensors 5a-d, and/or the electrical connector 6 may be printed onto the sensor strip, e.g. by using a conductive ink. Accordingly, the sensors and the electrical components are firmly attached to the sensor strip and remain functional even when the body moves or when the sensor strip is applied to a contoured or curved body part.

It is also conceivable that the plurality of sensors is adhesively attached to the adhesive sensor strip.

As illustrated in figures 1 and 2, the plurality of sensors 5a-c may be arranged in a linear sequence.

As used herein, the term "arranged in a linear sequence" means that the sensors are arranged along a straight line.

The distance between the sensors may vary depending on the size of the adhesive sensor strip and on the number of sensors used. The distance between one sensor and a neighboring sensor is typically at least 10 mm, e.g. from 10 to 40 mm.

The sensor strip has a top side and a bottom side. The plurality of sensors is typically arranged on the top side of the sensor strip. It is also conceivable that the plurality of sensors is arranged on the bottom side of the strip.

The plurality of sensors 5a-d is connected to the electrical connector 6 by an electrical circuit 21. The electrical connector 6 and the electrical circuit 21 are typically also arranged on the top side of the sensor strip.

As best illustrated in figures 1 and 3, at least the first portion 3 of the sensor strip 1 may comprise a plurality of perforations 7.

The perforations 7 secure exudate transfer from the incision or wound site to the overlying medical dressing 2.

The perforations 7 extend through the thickness of the sensor strip. In embodiments where the adhesive sensor strip comprises a polymeric film and an adhesive skin-contact layer, the perforations extend through the polymeric film and the adhesive skin-contact layer.

The perforations may be of various shapes and sizes. Preferably, the perforations are arranged in a predetermined, regular pattern. The perforations may have a diameter of from 0.5 mm to 8 mm, e.g. from 1 to 5 mm, preferably from 1 to 2 mm.

It is also conceivable that the second portion 4 comprises a plurality of perforations. Typically, the second portion is non-perforated. This facilitates printing the electrical conductor on the second portion of the sensor strip.

The first portion 3 further comprises a non-perforated area 22.

The electrical circuit may be arranged in the non-perforated area 22.

The non-perforated area 22 may represent the area surrounding the plurality of perforations 7. It is also conceivable that the non-perforated area 22 is an area extending in the lateral direction of the strip, e.g. below or above the plurality of sensors 5a-d.

The plurality of sensors 5a-d may also be arranged in the non-perforated area 22. Arranging the electrical circuit and/or the plurality of sensors 5a-d in the non-perforated area 22 allows for the electrical components of the sensor strip to be protected from direct moisture exposure. This may be beneficial to improve the performance and longevity of the sensors, as well as the transmission of data. It may also be beneficial to facilitate printing of the electrical components onto the sensor strip.

As best illustrated in figure 2, the second portion 4 may be divided into an intermediate section 8 and a terminal section 9; the intermediate section 8 being arranged between the first portion 3 and the terminal section 9 of the sensor strip 1, wherein the electrical connector 6 is arranged in the terminal section 9.

In figure 2, the portions of the strip 1 are illustrated with respect to an overlying medical dressing 2.

The medical dressing 2 is preferably a border dressing. As can be seen, the first portion 3 of the adhesive sensor strip 1 has a maximum lateral (x) extension corresponding to the maximum lateral (x) extension of the absorbent pad 17. In other words, the length of the first portion 3 generally corresponds to the length of the absorbent pad 17 of the medical dressing 2.

The intermediate section 8 has a maximum lateral (x) extension corresponding to the width of the border portion 18 of the medical dressing 2.

The maximum lateral (x) extension of the intermediate section 8 may be from 10 to 40 mm. Hence, the intermediate section 8 has a length corresponding to the width of a conventional border portion.

The border portion surrounds the absorbent pad. In other words, the border portion extends a distance beyond each peripheral edge of the absorbent pad.

The "width" of the border portion means the distance from a peripheral edge of the absorbent pad to the peripheral edge of the border portion (i.e. medical dressing dressing). This distance may vary along the peripheral edges of the medical dressing. Alternatively, it is the same around the whole periphery of the absorbent pad.

It is also conceivable that the backing layer, the absorbent pad, and the adhesive-skin contact layer are co-extensive in length and width. In such embodiments, the entire part of the second portion of the sensor strip extends beyond a peripheral dressing edge during use.

The maximum lateral (x) extension of the terminal section 9 may be in the range of from 20 to 50 mm, e.g. from 20 to 40 mm. These dimensions are suitable for housing the electrical connector 6.

The maximum lateral (x) extension of the first portion of the sensor strip is from 50 to 350 mm, e.g. from 100 to 300 mm.

The maximum lateral (x) extension of the third portion 20, if present, may be from 10 to 50 mm.

The medical dressing 2 is typically substantially rectangular (as illustrated in figures 1-4). However, the medical dressing is not limited to a rectangular shape, but any shape is conceivable, such as oval, square, round etc. It is also conceivable that the medical dressing has a shape adapted to fit with the sacrum or heel of a wearer.

As illustrated in figure 1, the medical dressing is defined by peripheral edges 2a-d. The peripheral edges 2a-d may be straight or curved. Typically, the medical dressing has a substantially rectangular shape. The corners of the medical dressing may be curved.

The medical dressing in figure 1 is defined by a first 2a and a second 2b lateral edge and a first 2c and a second 2d longitudinal edge.

The adhesive sensor strip is defined by first 1a and a second 1b lateral edge and a first 1c and a second 1d longitudinal edge.

A "lateral edge" of the medical dressing, adhesive sensor strip or absorbent pad means an edge extending in the longitudinal (y) direction, i.e. parallel to the y axis.

A "longitudinal edge" of the medical dressing, adhesive sensor strip, or absorbent pad means an edge extending the lateral (x) direction, i.e. parallel to the x axis.

The adhesive sensor strip is desirably thin and pliable such that it can stretch and conform to the movement of a wearer. As best illustrated in figure 3, the adhesive sensor strip 1 may comprise a polymeric film 10 and an adhesive skin-contact layer 11; the adhesive skin-contact layer 11 preferably comprising a silicone-based adhesive.

As mentioned hereinbefore, the adhesive sensor strip has a top side and a bottom side. The polymeric film 10 forms the top side of the sensor strip. The adhesive skin-contact layer 11 forms the bottom side of the sensor strip.

Typically, the plurality of sensors 5a-d, the electrical circuit 21 and the electrical connector 6 are arranged on the polymeric film 10 of the adhesive sensor strip.

The polymeric film 10 may comprise polyethylene, polyamide, polyester, and/or polyurethane. The thickness of the polymeric film may be in the range of from 15 to 50 µm, preferably from 20 to 40 µm.

Examples of suitable silicone-based adhesives include the two component RTV systems, such as Q72218 (Dow Corning), and SilGel 612 (Wacker Chemie AG), as well as NuSil silicone elastomers. In embodiments of the invention the adhesive may comprise a soft silicone gel having a softness (penetration) of from 8 to 22 mm, e.g. from 12 to 17 mm, as measured by a method based on ASTM D 937 and DIN 51580, the method being described in European Patent Application No 14194054.4.

The thickness of the adhesive skin-contact layer 11 is typically at least 20 µm. The thickness of the adhesive skin-contact layer may be from 50 to 250 µm.

As illustrated in figure 1, the adhesive sensor strip may further comprise a leakage-protective coating 12 arranged on at least the intermediate section 8 of the second portion 4.

The leakage-protective coating 12 is typically arranged on the top side of the sensor strip. In other words, the leakage-protective coating 12 may be arranged on the polymeric film 10 of the sensor strip 1.

The leakage-protective coating 12 is preferably arranged in the area where a border portion of a medical dressing is to be arranged on the adhesive sensor strip during use. The leakage-protective coating 12 serves to prevent exudate mitigating towards the border portion and causing detachment of the medical dressing 2 from the skin.

The leakage-protective coating 12 may comprise a formable polymeric material. For example, the leakage-protective coating may comprise silicone. The leakage-protective coating 12 may be adhesive or non-adhesive. Typically, the leakage-protective coating 12 is adhesive. For example, the leakage-protective coating 12 may be a silicone-based adhesive coating.

The leakage-protective coating 12 is preferably arranged at least along the longitudinal edges of the sensor strip in the intermediate section 9. The soft and formable coating (e.g. silicone-based adhesive coating) may then "fill the gaps" defined between the overlying dressing and the skin along the longitudinal edges of the sensor strip during use. This way, potential liquid reservoirs (through which exudate can migrate from the absorbent pad towards the border) are prevented from forming.

As illustrated in figure 1, the leakage-protective coating 12 may cover a small part of the first portion 3 and/or a small part of the terminal section 9.

In figure 3, the sensor strip further comprises a third portion 20 extending beyond a second peripheral edge 2b of the medical dressing. In such embodiments, it is beneficial to have a leakage-protective coating 12 in the area of the third portion 20 underlying the border portion of the medical dressing (see figure 3).

As best illustrated in figures 1 and 2, the adhesive sensor strip 1 may further comprise at least one printed marking 13 indicating where a peripheral edge of an overlying medical dressing 2 should be arranged on the sensor strip during use. In figures 1 and 2, the printed markings 13 are arrows indicating where a first lateral edge 2a of the medical dressing should be arranged on the sensor strip 1 during use.

The at least one printed marking 13 may comprise arrows, numbers, and/or printed instructions.

The printed marking(s) 13 guide the medical personnel to a correct arrangement of a medical dressing 2 on top of the sensor strip 1.

The at least one printed marking 13 may be arranged at the interface of the intermediate 8 and terminal 9 sections (see figure 2).

This is particularly beneficial in embodiments where the medical dressing is a border dressing.

According to another aspect, there is provided a medical dressing assembly 14 comprising the adhesive sensor strip 1 as described hereinbefore, and a medical dressing 2.

The medical dressing 2 is configured to be arranged on the first portion 3 of the sensor strip 1, and wherein at least the electrical connector 6 of the second portion 4 is arranged to extend beyond one of the peripheral edges of the medical dressing 2.

As illustrated in figure 1, the electrical connector 6 extends beyond a first lateral edge 2a of the medical dressing 2.

As used herein, the term "medical dressing assembly" means a combination of at least a medical dressing and an adhesive sensor strip as described hereinbefore. The medical dressing and the adhesive sensors strip are separate components which are assembled during use.

The medical dressing assembly may further comprise a separate processing device.

As used herein, the term "processing device" means a component comprising a power source, a processing unit, and means to communicate data with an electronic device, designed to receive, analyze, and transmit data from the sensors.

The processing device may be comprised in a patch as illustrated in figures 2 and 3.

The patch is typically flexible and formable.

The integration of the processing device within a patch ensures that the medical dressing assembly is compact, flexible, and user-friendly. The patch can be easily attached to the electrical connector of the sensor strip, which simplifies the handling for medical personnel. Furthermore, the medical dressing assembly is associated with less "bulk", and generally "softer" components, which is convenient for the patient.

The patch may be formed from conventional dressing materials or be formed from injection-molded foam, plastic, or other polymeric materials.

The patch 23 may be anchored to the electrical connector 6 on the second portion 4 of the sensor strip 1 as indicated by the dotted lines in figure 2. The patch 23 may be attached to the electrical connector 6 by adhesive or mechanical means.

In figure 2, the patch 23 is a border dressing. The patch 23 comprises a central portion 23a and a border portion 23b surrounding the central portion 23a. The central portion 23a of the patch 23 may comprise the processing device. The border portion 23b may be adhesive. Hence, the adhesive border portion 23b of the patch 23 may be attached to the skin of a patient during use.

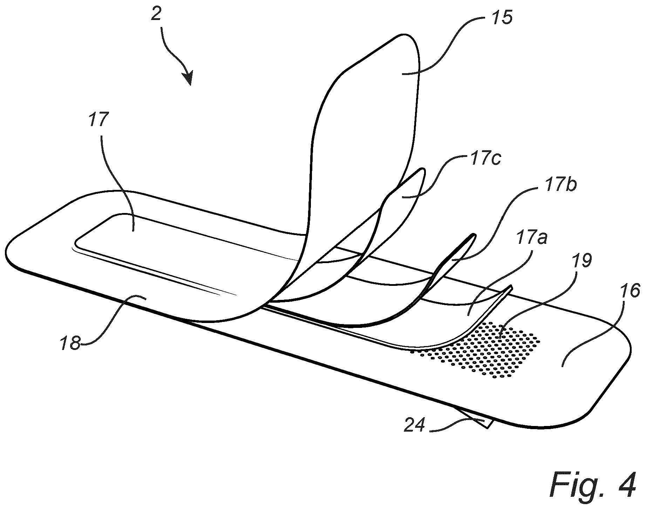

The medical dressing 2 of the present disclosure is preferably a medical dressing adapted for post-operative incisions. A preferred medical dressing 2 is illustrated in figure 4.

The medical dressing 2 may comprise a backing layer 15, an adhesive skin-contact layer 16, and an absorbent pad 17 arranged between the backing layer 15 and the adhesive skin-contact layer 16; the absorbent pad 17 being defined by pad edges, wherein the backing layer 15 and the adhesive skin-contact layer 16 are arranged to extend beyond the pad edges to form a border portion 18 surrounding the absorbent pad 17.

The "backing layer" is the top layer; i.e. the outermost layer of the medical dressing. The backing layer is a continuous layer and protects the layers of the dressing from entry of potential contaminants. The backing layer typically comprises a polymeric film, e.g. a polyurethane film. The thickness of the backing layer may be in the range of from 15 to 50 µm, preferably from 20 to 40 µm.

The "adhesive skin-contact layer" of the medical dressing is configured to detachably adhere the dressing to a dermal surface. In other words, the adhesive skin-contact layer is configured to contact the skin or the wound or incision. In the context of the present disclosure, the adhesive skin contact layer is also arranged in contact with the adhesive sensor strip.

The adhesive skin-contact layer 16 may comprise a polymeric film and a silicone-based adhesive coating; the silicone-based adhesive coating being arranged to contact the skin of a wearer during use.

The adhesive skin-contact layer 16 may have the same general construction as the adhesive sensor strip 1. The polymeric film may comprise polyethylene, polyamide, polyester or polyurethane. The thickness of the polymeric film may be from 15 to 100 µm, e.g. from 20 to 80 µm, preferably from 20 to 60 µm.

The silicone-based adhesive coating may be the same as that described hereinbefore with respect to the silicone-based adhesive of the adhesive sensor strip.

The medical dressing has a lateral (x) extension and a longitudinal (y) extension. As illustrated in e.g. figures 1 and 2 the absorbent pad 17 has a maximum lateral (x) extension corresponding to the maximum lateral (x) extension of the first portion 3 of the sensor strip 1, wherein the border portion 18 has a width corresponding to the maximum lateral (x) extension of the intermediate section 8 of the second portion 4 of the sensor strip 1.

As can be seen in figure 4, the adhesive skin-contact layer 16 may comprise a plurality of perforations 19 in the area underlying the absorbent pad 17, but is void of perforations in the area forming the border portion 18.

This construction is beneficial to improve the adherence of the dressing onto the skin; i.e. to enhance the wear time of the medical dressing. It is also beneficial to prevent leakage of exudate along the length of the sensor strip (particularly from the absorbent pad towards the border portion), as described hereinbefore.

In the context of the present disclosure, the "absorbent pad" may comprise one or a plurality of pad-forming layers. Typically, the absorbent pad comprises more than one layer.

The absorbent pad may e.g. comprise an absorbent polyurethane foam layer.

The absorbent pad may further comprise a superabsorbent layer, i.e. a layer comprising a superabsorbent material. The superabsorbent material may be superabsorbent polymer (SAP) particles or superabsorbent fibres (SAF). The superabsorbent material is capable of handling large amounts of wound exudate.

The absorbent pad may also comprise a liquid distribution layer. The liquid distribution layer may comprise any material having the ability to distribute the exudate in an efficient manner. For example, the liquid distribution layer may comprise a nonwoven material.

A layered pad construction prevents accumulation of body liquids close to the skin and improves the overall liquid handling of the medical dressing.

For example, the absorbent pad illustrated in figure 4 may comprise a first absorbent layer 17a, a liquid distribution layer 17b and a second absorbent layer 17c. Typically, the liquid distribution layer 17b is arranged between the first 17a and the second 17c absorbent layers, wherein the first absorbent layer 17a is the lowermost layer of the absorbent pad.

The first absorbent layer 17a may comprise a foam. Suitable foam materials for use in the first absorbent layer 17a include, but are not limited to polyurethane foams.

The second absorbent layer 17c may be a superabsorbent layer. Accordingly, the second absorbent layer may comprise superabsorbent polymers (SAP) or superabsorbent fibers (SAF).

The liquid distribution layer 17b may comprise any material having the ability to distribute the exudate in an efficient manner. For example, the liquid distribution layer 17b may comprise a nonwoven material. A nonwoven imparts an appropriately balanced rigidity to the layer and to the dressing as such. It may also efficiently distribute and spread liquid absorbed by the first absorbent layer 17a such that it can be evaporated through the backing layer 15 over a large surface. For example, the nonwoven may comprise viscose, polyester or blends thereof.

The layers can be joined by adhesion, lamination, using e.g. pressure and heat.

The absorbent pad 17 may comprise additional layers, such as liquid transport layers, various combinations of foam and nonwoven layers laminated together.

In an infected wound, the exudate production is typically large. A medical dressing having the construction as explained hereinabove is suitable for handling large amounts of exudate and for preventing maceration of the skin surrounding the wound. Thus, the medical dressing is particularly suited for infection prevention.

The medical dressing 2 typically further comprises a release liner detachably attached to the adhesive skin-contact layer 16. The release liner may comprise one or a plurality of release liner portions and is configured to cover the entire surface of the adhesive skin-contact layer 16. In figure 2, one of the release liner portions is shown and is denoted 24.

As mentioned hereinbefore, the medical dressing 2 is preferably void of any sensors and of any electrical components.

According to another aspect, there is provided a kit comprising the adhesive sensor strip as described hereinbefore and a processing device. The processing device is preferably comprised in a patch, as described hereinbefore.

The kit may further comprise at least one medical dressing.

The components of the kit may be provided in the same package.

Terms, definitions and embodiments of all aspects of the present disclosure apply mutatis mutandis to the other aspects of the present disclosure.

Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

## Claims

1. An adhesive sensor strip (1) for use in conjunction with a medical dressing (2), wherein said sensor strip (1) has a lateral (x) and a longitudinal (y) extension; said lateral (x) extension being larger than said longitudinal (y) extension, wherein said sensor strip (1) comprises a first portion (3) and a second portion (4); the maximum lateral (x) extension of said first portion (3) being from 50 to 350 mm, wherein the maximum lateral (x) extension of said second portion (4) is from 15 to 100 mm, wherein said first portion (3) comprises a plurality of sensors (5a-d) configured to monitor at least one parameter indicative of infection, and wherein said second portion (4) comprises an electrical connector (6) being connected to said plurality of sensors (5a-c) and being configured for connection with a separate processing device.

2. The adhesive sensor strip (1) according to claim 1, wherein said second portion (4) is void of sensors.

3. The adhesive sensor strip (1) according to claim 1 or claim 2, wherein said plurality of sensors (5a-d) is selected from the group consisting of temperature sensors, pH sensors, humidity sensors and/or biochemical sensors, preferably wherein said plurality of sensors (5a-d) is temperature sensors.

4. The adhesive sensor strip (1) according to claim 1, wherein said plurality of sensors (5a-c) is arranged in a linear sequence.

5. The adhesive sensor strip (1) according to any one of the preceding claims, wherein at least said first portion (3) of said sensor strip (1) comprises a plurality of perforations (7).

6. The adhesive sensor strip (1) according to any one of the preceding claims, wherein said second portion (4) is divided into an intermediate section (8) and a terminal section (9); said intermediate section (8) being arranged between said first portion (3) and said terminal section (9) of said sensor strip (1), wherein said electrical connector (6) is arranged in said terminal section (9).

7. The adhesive sensor strip (1) according to any one of the preceding claims, wherein said adhesive sensor strip (1) comprises a polymeric film (10) and an adhesive skin-contact layer (11); said adhesive skin-contact layer (11) preferably comprising a silicone-based adhesive.

8. The adhesive sensor strip (1) according to any one of the preceding claims when dependent on claim 6, wherein said sensor strip (1) further comprises a leakage-protective coating (12) arranged on at least said intermediate section (10) of said second portion (4).

9. The adhesive sensor strip (1) according to any one of the preceding claims, further comprising at least one printed marking (13) indicating where a peripheral edge of said medical dressing (2) should be arranged on said sensor strip (1) during use.

10. The adhesive sensor strip (1) according to claim 9 when dependent on claim 6, wherein said at least one printed marking (13) is arranged at the interface of said intermediate (8) and terminal (9) sections.

11. A medical dressing assembly (14) comprising the adhesive sensor strip (1) according to any one of claims 1-10, and a medical dressing (2), wherein said medical dressing (2) is arranged on top of said first portion (3) of said sensor strip (1) during use; said medical dressing being defined by peripheral edges, and wherein at least said electrical connector (6) of said second portion (4) is arranged to extend beyond one of said peripheral edges of said medical dressing (2).

12. The medical dressing assembly (14) according to claim 11, wherein said medical dressing (2) comprises a backing layer (15), an adhesive skin-contact layer (16), and an absorbent pad (17) arranged between said backing layer (15) and said adhesive skin-contact layer (16); said absorbent pad (17) being defined by pad edges, wherein said backing layer (15) and said adhesive skin-contact layer (16) are arranged to extend beyond said pad edges to form a border portion (18) surrounding said absorbent pad (17).

13. The medical dressing assembly (14) according to claim 12 when dependent on claim 6, wherein said medical dressing has a lateral (x) extension and a longitudinal (y) extension; said absorbent pad (17) having a maximum lateral (x) extension corresponding to the maximum lateral (x) extension of said first portion (3) of said sensor strip (1), wherein said border portion has a width corresponding to the maximum lateral (x) extension of said intermediate section (8) of said second portion (4) of said sensor strip (1).

14. The medical dressing assembly (14) according to claim 12 or claim 13, wherein said adhesive skin-contact layer (16) comprises a plurality of perforations (19) in the area underlying said absorbent pad (17) but is void of perforations in the area forming said border portion (18).

15. The medical dressing assembly (14) according to any one of claims 11-14, further comprising a processing device configured for connection with said electrical connector (6) of said adhesive sensor strip (1).

16. The medical dressing assembly (14) according to any one of claims 11-15, wherein said medical dressing (2) is void of any sensors and of any electrical components.
